# EUROPÄISCHE PATENTSCHRIFT

(11) **EP 3 368 017 B1**
(45) Veröffentlichungstag und Bekanntmachung des Hinweises auf die Patenterteilung: **06.11.2019**
(21) Anmeldenummer: 16787790.1
(22) Anmeldetag: 21.10.2016
(51) Int. Cl.: A61K 9/20, A61K 9/28, A61K 31/606

(54) **OPTIMIERTE MESALAZINHALTIGE HOCHDOSISTABLETTE**
OPTIMIZED HIGH-DOSE TABLET OF MESALAZINE
COMPRIMÉ OPTIMISÉ A UNE HAUTE DOSE CONTENANT DE LA MESALAZINE

(30) Priorität: 30.10.2015 EP 15192269
(43) Veröffentlichungstag der Anmeldung: 05.09.2018
(73) Patentinhaber: Dr. Falk Pharma GmbH, 79108 Freiburg (DE)
(72) Erfinder: WILHELM, Rudolph, 76476 Bischweier (DE); PRÖLS, Markus, 79100 Freiburg/Breisgau (DE); GREINWALD, Roland, 79341 Kenzingen (DE); NACAK, Tanju, 79288 Gottenheim (DE)
(74) Vertreter: Keller, Günter
(86) Internationale Anmeldenummer: PCT/EP2016/075427
(87) Internationale Veröffentlichungsnummer: WO 2017/072050

(56) Entgegenhaltungen:
- EP-A1- 2 425 826
- WO-A1-00/44353
- WO-A1-2011/045775
- WO-A1-2012/089677
- WO-A1-2015/062640
- WO-A2-2009/047802
- CA-A1- 2 444 814
- US-A1- 2015 196 518

## Beschreibung

Die chronisch entzündlichen Darmerkrankungen (CED) sind in Schüben verlaufende, destruierende Entzündungen des Intestinaltrakts. Hierunter werden die beiden häufigsten Formen Morbus Crohn und Colitis ulcerosa sowie die seltenere kollagene Colitis, die lymphozytäre Colitis und die atypische mikroskopische Colitis zusammengefasst. Morbus Crohn und Colitis ulcerosa unterscheiden sich voneinander in ihrem Verteilungsmuster sowie ihrem makroskopischen und histologischen Bild [Dignass et al. (2012) Journal of Crohn's and Colitis 6, 965-990].

Der Morbus Crohn kann segmental in allen Abschnitten vom Ösophagus bis zum Rektum auftreten und befällt alle Wandschichten. Typische Symptome sind krampfartige Schmerzen, Diarrhoe, Fieber und Gewichtsverlust. Die Colitis ulcerosa beginnt im Rektum, kann sich von hier auf alle Abschnitte des Colons ausdehnen und befällt zirkulär nur die Mukosa. Die Patienten leiden unter blutigen Diarrhöen, krampfartigen Schmerzen, Appetitlosigkeit und Gewichtsverlust. Die Ätiologie der chronisch entzündlichen Darmerkrankungen ist noch ungeklärt, sodass kausale Behandlungsmöglichkeiten fehlen.

Zur Therapie und Rezidivprophylaxe chronisch-entzündlicher Darmerkrankungen wie Colitis ulcerosa und Morbus Crohn werden Mesalazin-freisetzende Medikamente eingesetzt. Mesalazin ist der internationale Freiname für 5-Aminosalicylsäure. Auch wenn der genaue Wirkungsmechanismus noch nicht eindeutig geklärt ist, kann die therapeutische Wirksamkeit der Substanz auf eine lokale Entzündungshemmung an der Darmwand zurückgeführt werden. Mesalazin wird als Standardbehandlung der ersten Wahl (first-line treatment) oral und/oder rektal angewendet. Für die orale Behandlung des akuten Schubs der Colitis ulcerosa sowie zur Rezidivpophylaxe hat sich eine Hochdosistherapie mit Mesalazin als wirksam erwiesen, wobei eine Tagesdosis von 3 g, einmal täglich oder verteilt auf 3 Einzelgaben (morgens, mittags, abends), verabreicht wird.

Zur Vermeidung einer Wirkstoffresorption in den oberen Dünndarmabschnitten nach oraler Applikation sowie zum gezielten Transport von Mesalazin an den gewünschten Wirkort im Darm müssen besondere galenische Formulierungen bereitgestellt werden. Um dieses Ziel zu erreichen, kann die Einzeldosis des Wirkstoffs entweder in Form einer monolithischen, kompakten Arzneiform, wie zum Beispiel einer magensaftresistent überzogenen Tablette oder einer Tablette mit modifizierter Wirkstofffreisetzung, formuliert werden, oder in Gestalt einer multipartikulären abgeteilten Arzneiform, wie zum Beispiel als magensaftresistent überzogene Granulate/Pellets oder Granulate/Pellets mit modifizierter Wirkstofffreisetzung. Diese Darreichungsformen sind bekannt, vermeiden eine vorzeitigte Wirkstofffreisetzung und ermöglichen stattdessen die verzögerte, kontinuierliche oder diskontinuierliche Freisetzung der Arzneistoffdosis sowie das Verbringen von Mesalazin an den gewünschten Zielort. Dabei hat die monolithische Arzneiform den Vorteil, dass das Volumen bei gleicher Dosis und Zusammensetzung in der Regel kleiner ist als das einer multipartikulären Arzneiform. Tabletten sind daher für viele Patienten einfacher zu schlucken als entsprechende multipartikuläre Formen mit derselben Dosis.

Neben diesen galenischen Anforderungen ist die korrekte Einnahme des Medikaments sowie die genaue Befolgung der Dosierungsanleitung und der Art und Dauer der Anwendung durch den Patienten eine wesentliche Voraussetzung für die Wirksamkeit einer Mesalazin-Behandlung. Die Therapietreue ("Compliance") zeigt sich darin, wie genau und wie lange sich der Patient an das verordnete Dosierungsschema hält. Mangelnde Therapietreue ("Non-Compliance") durch den Patienten beeinflusst das Therapieergebnis bis hin zur Unwirksamkeit.

Die Weltgesundheitsorganisation ("WHO") beschreibt neben sozialen/ökonomischen, systembedingten und krankheitsbedingten Faktoren therapie- und patientenbedingte Phänomene als Ursachen für eine mangelnde Therapietreue. Als Ausprägungen von therapie- und patientenbedingten Faktoren können unter anderem die Komplexität des Behandlungsregimes und die Behandlungsdauer, sowie die Motivation, die Erwartungen und die Vergesslichkeit des Patienten genannt werden. Eine wesentliche Maßnahme zur Verbesserung der Compliance ist daher die Vereinfachung der medikamentösen Therapie.

Ungeeignete Dosierungsschemata für die orale Hochdosistherapie von entzündlichen Darmerkrankungen mit Mesalazin können den Alltag und damit die Lebensqualität der betroffenen Patienten stark beeinträchtigen. Dies trifft vor allem für Patienten zu, bei denen nach einem Ansprechen der Therapie das Wiederauftreten der Erkrankung verhindert werden soll. Die Therapietreue kann durch die Gabe patientenfreundlicher Darreichungsformen, durch die Verringerung der Anzahl an einzunehmenden Medikamenten sowie durch ein vereinfachtes Dosierungsschema deutlich verbessert werden. In diesem Zusammenhang stellt die tägliche und korrekte Einnahme von hohen Tagesdosen Mesalazin eine große Herausforderung für den Patienten dar.

Im Stand der Technik sind verschiedene Lösungen für die Anwendung oraler Mesalazinpräparate beschrieben. Etabliert ist die therapeutische Anwendung magensaftresistent überzogener Tabletten von Mesalazin mit relativ geringen Wirkstoffmengen, wie zum Beispiel Salofalk® 250 mg /-500 mg magensaftresistente Tabletten (EP 0 083 775), Claversal® 250 mg/-500 mg magensaftresistente Tabletten oder Asacol® 400 mg magensaftresistente Tabletten. Diese Darreichungsformen besitzen Filmüberzüge auf Basis von polymeren Substanzen mit pH-abhängigen Löslichkeitseigenschaften. Je nach Auflösungs-pH-Wert sowie der Menge an aufgetragenem Filmbildner kann ein bestimmter Abschnitt des Darms für den Beginn der Mesalazin-Freisetzung angesteuert werden. Nach der vollständigen Auflösung des Filmüberzugs im Dünndarm ist von einer raschen Freisetzung des Mesalazins aus dem zurückbleibenden und anschließend zerfallenden Tablettenkern auszugehen. Die Arzneistofffreisetzung erfolgt damit verzögert aber nicht modifiziert.

Neuere Mesalazin enthaltende orale Darreichungsformen beschreiben auch Hochdosisformulierungen, die allerdings den Nachteil einer sehr komplexen pharmazeutischen Technologie haben. So werden multipartikuläre Formulierungen in Form von Pellets beschrieben (EP 0 977 557). In der EP 0 977 557 wird eine oral verabreichbare Pellet-Formulierung mit einem kontrollierten Freigabeprofil beschrieben. Die kontrollierte Freisetzung wird hierbei durch eine komplexe Polymermatrix in der der Wirkstoff im Kern der Pellets vorliegt und magensaftresistenten Filmüberzügen erreicht. Die Verwendung von Sachets ermöglicht die Darreichung von mehr als 1000 mg Mesalazin-Pellets pro Sachet. Die WO 2004/093884 beschreibt die Darreichung einer Mesalazin-Formulierung mit verzögerter Freisetzung als Sachet in einer Dosierung zwischen 500 mg bis 10 g pro Sachet. Auch in Tablettenform sind lediglich komplexe pharmazeutische Technologien bekannt. In der EP 1 198 226 wird ebenso eine innere Matrix beschrieben, in welcher der aktive Bestandteil zumindest teilweise eingeschlossen ist. Zusätzlich enthält diese orale Darreichungsform eine äußere hydrophile Matrix und fakultativ weitere, andere Trägerstoffe.

Eine weitere Hochdosisformulierung beinhaltet einen Matrixkern mit einer definierten Viskosität, wobei diese hydrophile Matrix in einer Menge von 1 bis 20% bezogen auf das Gesamtgewicht der Tablette vorhanden sein muss (EP 2 621 477).

In der WO 2011/045775 werden Tabletten mit einem verhältnismäßig hohen Gehalt an Mesalamin (1200 mg pro Tablette) offenbart. Bei der Herstellung wird granuliertes Mesalamin mit matrixbildenden Stoffen wie Carbopol vermischt und anschließend verpresst. Diese Tabletten werden mit einer einzigen Schicht eines enterischen Überzugsmittels versehen.

Die WO 00/44353 beschreibt pharmazeutische Zusammensetzungen zur langsamen Freisetzung verschiedenster Wirkstoffe im Magen-Darm-Trakt. Dabei werden mehrere wirkstoffhaltige Teilchen mit einem Magen- und Darmsaft-unlöslichen Material überzogen, wobei die einzelnen Teilchen nach Umhüllung mit Überzugsmitteln wie Eudragit® RS oder Eudragit® NE miteinander verpresst werden. Dabei stellt der wirkstoffhaltige Kern ein homogenes Gemisch umfassend den Wirkstoff und ein im Magen- und Darmsaft unlösliches Polymer dar.

Die WO 2015/062640 offenbart eine Isolationsschicht für die beschleunigte Wirkstofffreisetzung im Darm für pharmazeutische Formulierungen mit verzögerter Freisetzung. Die dort beschriebenen Tabletten beinhalten einen Kern mit dem pharmazeutischen Wirkstoff, eine Isolierungsschicht, die eine beschleunigte Freisetzung des Wirkstoffes im Darm bewirkt, und, die den Kern bedeckt, sowie eine äußere Beschichtung, die wiederum aus einer inneren und einer äußeren Schicht besteht. Diese äußere und innere Schicht der äußeren Umhüllung umfassen Polymermaterialien, die sich bei einem pH-Wert über 6 auflösen.

Die WO 2009/047802 beschreibt pharmazeutische Formulierungen für verschiedenste Wirkstoffe, u.a. 5-Aminosalicylsäure oder deren Metaboliten. Diese werden in einer bioadhäsiven Formulierung verabreicht. Die verzögerte Freisetzung wird dadurch bewirkt, dass der Wirkstoff mit einem in Magensaft unlöslichen Polymer vermischt und anschließend verpresst wird. Diese Matrixtablette wird anschließend mit einer enterischen Beschichtung überzogen.

Die US 2015/196518 A1 offenbart Caplets, die 800 mg Mesalamin enthalten. Zunächst wird eine Vormischung hergestellt, die granuliert, getrocknet und zu Caplets verpresst wird.

Diese Caplets werden mit einer enterischen Beschichtung versehen und in Gelatinekapseln verbracht, die verhältnismäßig groß sind und weniger Wirkstoff beinhalten.

Aufgabe der vorliegenden Erfindung ist es, eine stabile Hochdosistablette von Mesalazin bereitzustellen, die durch eine optimierte Rezeptur die Verabreichung von 1000 mg Wirkstoff je Einheit ermöglicht. Durch die gewählte Formgebung und Größe kann die Tablette problemlos geschluckt werden, so dass mit einem vereinfachten Dosierungsschema die gleiche therapeutische Wirksamkeit wie z.B. mit der Einnahme der doppelten Anzahl an Salofalk® 500 mg magensaftresistenten Tabletten erzielt werden kann. Die medikamentöse Therapie wird hierdurch deutlich vereinfacht, und die regelmäßige Medikamenteneinnahme wird sichergestellt. Alle Eigenschaften der vorliegenden Erfindung steigern daher die Akzeptanz der Darreichungsform bei den Patienten und führen zu einer erhöhten Therapietreue.

Ein weiterer Aspekt der vorliegenden Erfindung ist, dass die Hochdosistablette von Mesalazin von den Patienten gut angenommen wird. Hierbei wesentlich ist, dass die an sich relativ große Tablette gut und problemlos geschluckt werden kann. Andererseits soll die erfindungsgemäße Hochdosistablette ein Freisetzungsprofil des Wirkstoffes aufweisen, das dem Freisetzungsprofil von zwei kleineren Tabletten entspricht. Es soll also beispielsweise eine Hochdosistablette von Mesalazin mit 1000 mg Wirkstoff ein Freisetzungsprofil aufweisen, das dem Wirkstoffprofil von zwei Tabletten mit je 500 mg Wirkstoff entspricht.

Weiterhin soll - um die Belastung des Patienten zu reduzieren - der relative Anteil an Hilfsstoffen und Beschichtungsmaterialien möglichst gering gehalten werden. Hilfsstoffe und Beschichtungsmaterialien können - zumindest von einigen Patienten - nur schlecht vertragen werden, deshalb sollte der Anteil möglichst gering gehalten werden.

Die vorliegende Erfindung betrifft daher eine orale magensaftresistente Hochdosistablette, umfassend als aktiven Bestandteil 700 mg bis 1200 mg Mesalazin oder ein pharmazeutisch verträgliches Salz davon und zumindest einen Hilfsstoff, wobei die Masse der Hochdosistablette maximal 35 % und besonders bevorzugt maximal 25 % höher als die Masse des aktiven Bestandteils ist und, wobei der zumindest eine Hilfsstoff im Kern keine Matrix-bildende Bestandteile enthält. Die erfindungsgemäße Formulierung ist keine Matrixtablette, bei der die Freisetzung des Wirkstoffs über die im Tablettenkern befindliche Matrix gesteuert wird.

Unter magensaftresistent wird vorliegend verstanden, dass die Hochdosistablette die Anforderungen an magensaftresistsente Tabletten gemäß den Vorgaben der Monographien zu Darreichungsformen des europäischen Arzneibuches erfüllt.

Unter Hochdosistablette wird vorliegend eine Tablette mit einer hohen Dosis an aktivem Bestandteil verstanden. In der vorliegenden Erfindung wird unter einer hohen Dosis eine Menge von etwa 1000 mg an aktivem Bestandteil verstanden. Unter etwa 1000 mg wird ein Bereich von 980 mg bis 1020 mg an aktivem Bestandteil, nämlich Mesalazin, verstanden.

Die erfindungsgemäße Hochdosistablette enthält etwa 1000 mg Mesalazin. Damit kann eine Tagesdosis von 3 g Mesalazin entweder durch die einmalige Gabe von drei Hochdosistabletten oder durch die dreimal tägliche Anwendung (morgens, mittags, abends) von je einer Hochdosistablette realisiert werden.

Der zumindest eine Hilfsstoff wird aus den üblichen pharmazeutisch verträglichen Hilfsstoffen zur Granulierung und Tablettenherstellung ausgewählt. Dieser zumindest eine Hilfsstoff bildet mit dem Wirkstoff den Tablettenkern. Der zumindest eine Hilfsstoff besteht ausschließlich aus Polyvinylpyrrolidon, besonders bevorzugt Povidon K25. So wird zum Beispiel der aktive Bestandteil Mesalazin mit Hilfe einer Bindemittellösung bestehend aus Wasser und Povidon K25 durch Feuchtgranulierung in ein Wirkstoffgranulat überführt. Bevorzugt ist Povidon K25 hierbei in einer Menge von 50 mg bis 80 mg, besonders bevorzugt von 66,5 mg bis 73,5 mg pro 1000 mg Mesalazin in der Hochdosistablette enthalten.

In einer weiteren Ausführungsform kann die erfindungsgemäße Hochdosistablette neben dem Hilfsstoff, welcher bevorzugt Povidon K25 ist, weitere Hilfsstoffe ausgewählt aus Trockenbindemittel, Zerfallshilfsmittel, Fließregulierungsmittel und Schmiermittel enthalten. Derartige weitere Hilfsstoffe werden bevorzugt in einer Menge von maximal 180 mg pro 1000 mg Mesalazin zugegeben.

Trockenbindemittel werden zum Beispiel ausgewählt aus der Gruppe umfassend Calciumphosphate, Lactose, Stärke, Cellulose, Hexite, synthetische Polymere. Besonders bevorzugt ist hierbei mikrokristalline Cellulose. Bevorzugt ist mikrokristalline Cellulose hierbei in einer Menge von 95,0 mg bis 105,0 mg pro 1000 mg Mesalazin Hochdosistablette enthalten.

Zerfallshilfsmittel werden zum Beispiel ausgewählt aus der Gruppe umfassend Stärken, Cellulose, Cellulosederivate, synthetische Polymere. Besonders bevorzugt ist hierbei Croscarmellose-Natrium. Bevorzugt ist Croscarmellose-Natrium hierbei in einer Menge von 57,0 mg bis 63,0 mg pro 1000 mg Mesalazin Hochdosistablette enthalten.

Fließregulierungsmittel werden zum Beispiel ausgewählt aus der Gruppe umfassend Stärke, Metallseifen, wasserfreies hochdisperses Siliziumdioxid. Besonders bevorzugt ist hierbei wasserfreies hochdisperses Siliziumdioxid. Bevorzugt ist wasserfreies hochdisperses Siliziumdioxid hierbei in einer Menge von 4,75 mg bis 5,25 mg pro 1000 mg Mesalazin Hochdosistablette enthalten.

Schmiermittel werden zum Beispiel ausgewählt aus der Gruppe umfassend Metallseifen, Fettalkohole, Talk, höhermolekulare Polyethylenglykole. Besonders bevorzugt ist hierbei Calciumstearat. Bevorzugt ist Calciumstearat hierbei in einer Menge von 12,4 mg bis 13,7 mg pro 1000 mg Mesalazin Hochdosistablette enthalten.

Desweiteren enthält der zumindest eine Hilfsstoff keine Matrix-bildenden Bestandteile. Unter Matrix-bildenden Bestandteilen werden vorliegend sämtliche Bestandteile verstanden, welche den aktiven Bestandteil in ein Gerüst einbetten und so die Freisetzung des aktiven Bestandteils beeinflussen. Beispiele für Matrix-bildende Bestandteile sind, ohne abschließend zu sein, Cellulosederivate, wie zum Beispiel Ethylcellulose, Hydroxypropylmethylcellulose, Wachse, Polyvinylacetat, Polymere und Copolymere von Acrylaten und Methacrylaten, wie zum Beispiel Eudragit® der Typen RL, RS oder NE. Als Matrix-bindende Bestandteile werden im Sinn der vorliegenden Erfindung insbesondere solche Zusatzstoffe verstanden, die im Tablettenkern vorhanden sind und eine Verzögerung der Freisetzung des Wirkstoffs bewirken.

Die Hochdosisfilmtablette gemäß der vorliegenden Erfindung ist dadurch gekennzeichnet, dass die Masse der Hochdosisfilmtablette maximal 35 % höher als die Masse des aktiven Bestandteils ist. Dies bedeutet, dass zum Beispiel eine Tablette mit einer Masse an aktivem Bestandteil von 1000 mg maximal eine Gesamtmasse von 1350 mg aufweist.

In einer Ausführungsform ist die Masse des Tablettenkerns der Hochdosistablette maximal 35 % höher und besonders bevorzugt maximal 30 % und ganz besonders bevorzugt maximal 25 % höher als die Masse des aktiven Bestandteils.

In einer weiteren Ausführungsform ist der Anteil des zumindest einen Hilfsstoffes maximal 35 Gew.-% und besonders bevorzugt maximal 20 Gew.-% bezogen auf das Gesamtgewicht der Hochdosistablette.

Die Entwicklung einer 1000 mg Hochdosistablette auf Basis der Rezeptur der Salofalk® 500 mg magensaftresistenten Tabletten über eine einfache Verdoppelung der Bestandteile ist dabei nur eine theoretische Option, da dieses Vorgehen zu nicht mehr schluckbaren Formlingen mit einer Masse von ca. 1,7 g führen würde. In diesem Fall ist die Masse der Tablette 70% höher als der reine Wirkstoffanteil.

Im Gegensatz hierzu kommt die erfindungsgemäße Hochdosistablette, die äquivalente biopharmazeutische und therapeutische Eigenschaften mit Salofalk® 500 mg magensaftresistenten Tabletten aufweist, aber mit einem Hilfsstoffanteil von nur ca. 25% aus, so dass die Masse der Tablette maximal 35% höher als der reine Wirkstoffanteil ist. Wie in Beispiel 1 aufgeführt, hat die erfindungsgemäße 1000 mg-Hochdosistablette nur noch eine Masse von etwa 1,3 g. Unter Beibehaltung der Funktionalität sind diese Formlinge damit deutlich kleiner und für eine orale Anwendung viel besser geeignet als Tablettenrezepturen, hergestellt über eine einfache Verdopplung. Damit verbunden ist eine gesteigerte Patientenakzeptanz.

Die erfindungsgemäße Hochdosistablette besteht aus einem Tablettenkern mit optimierten Abmessungen bezüglich Form und Größe und in einer Ausführungsform zumindest einem Filmüberzug. Der Tablettenkern wird durch Verpressen einer Pulvermischung hergestellt. Sie enthält das Wirkstoffgranulat in einer Abmischung mit zumindest einem Hilfsstoff. Die gesamte Wirkstoffmenge je Tablette ist dabei im Granulat verarbeitet.

Im Gegensatz zu dem zumindest einen Hilfsstoff kann der zumindest eine Filmüberzug Matrix-bildende Bestandteile enthalten. Der zumindest eine Filmüberzug kann in mehreren Schichten aufgetragen werden. Besonders bevorzugt ist die Auftragung von 2, 3 oder mehr Schichten. Diese Schichten können sowohl aus denselben, als auch unterschiedlichen filmbildenden Substanzen bestehen. Bevorzugt werden erfindungsgemäß solche Filmüberzüge eingesetzt, die keine Matrix bilden, sondern sich *in vivo* auflösen. Der zumindest eine Filmüberzug ist ausgewählt aus der Liste von filmbildenden Substanzen bestehend aus Hypromellose, Celluloseacetatphthalat, Hydroxypropylmethylcellulosephthalat, Hydroxypropylmethylcelluloseacetatsuccinat, Polyvinylacetatphthalat, Schellack, anionische Copolymere der Methacrylsäure und ihrer Ester vom Eudragit®-Typ, wie zum Beispiel Eudragit® L, Eudragit® S, oder Mischungen davon.

In einer bevorzugten Ausführungsform besteht der Filmüberzug aus einer Grundierschicht, sowie zwei Schichten, die der Sicherstellung der verzögerten Wirkstofffreisetzung dienen. Zum Beispiel besteht die Grundierschicht aus Hypromellose und die Schichten, die der Sicherstellung der verzögerten Wirkstofffreisetzung dienen, aus Eudragit® L und Eudragit® S. Die Grundierschicht ist, was die Freisetzung des Wirkstoffes in vivo angeht, neutral. Die Grundierschicht beschleunigt weder allein noch in Kombination mit der äußeren Umhüllungsschicht die Freisetzung des Wirkstoffes.

In einer besonders bevorzugten Ausführungsform weist die erfindungsgemäße Hochdosistablette eine verhältnismäßig dünne "Grundierschicht" aus Hypromellose (Hydroxypropylmethylcellulose) auf. Ein Vorteil dieser Grundierschicht besteht darin, dass die darauf aufgebrachte enterische Beschichtung besser und gleichmäßiger aufgetragen werden kann und, dass dadurch auch dünnere Schichten der aus ein oder zwei Schichten bestehenden enterischen Beschichtung aufgebracht werden können. Dies führt letztendlich dazu, dass bezogen auf den Wirkstoffgehalt weniger Beschichtungsmaterial aufgebracht wird und dabei dennoch das gewünschte Freisetzungsprofil erzielt werden kann.

In einer weiteren bevorzugten Ausführungsform besteht der Filmüberzug aus zwei Schichten, die der Sicherstellung der verzögerten Wirkstofffreisetzung dienen. Zum Beispiel bestehen die zwei Schichten aus Eudragit® L oder Eudragit® S oder Mischungen aus Eudragit® S und L.

In einer bevorzugten Ausführungsform wird das Wirkstoffgranulat durch Feuchtgranulierung von Mesalazin mit einer Bindemittellösung hergestellt, die aus Wasser als Lösungsmittel und einem wasserlöslichen Polymer, bevorzugt Povidon K25, als Bindemittel besteht. Die zugesetzte Menge an Povidon K25 entspricht dabei 66,5 mg bis 73,5 mg je Tablette. Nach dem Feuchtsieben und Trocknen des Wirkstoffgranulats werden Hilfsstoffe zur Verbesserung der Zerfallseigenschaft, bevorzugt Croscarmellose-Natrium in einer Menge von 57,0 mg bis 63,0 mg je Tablette, Trockenbindemittel, bevorzugt mikrokristalline Cellulose in einer Menge von 95,0 mg bis 105,0 mg je Tablette, Fließregulierungsmittel, bevorzugt wasserfreies hochdisperses Siliziumdioxid in einer Menge von 4,75 mg bis 5,25 mg je Tablette, und Schmiermittel, bevorzugt Calciumstearat in einer Menge von 12,4 mg bis 13,7 mg je Tablette, zugesetzt.

Die aus Wirkstoffgranulat und weiteren Hilfsstoffen bestehende Endmischung lässt sich hervorragend verformen. Durch das Verpressen der abgemischten Granulate resultieren Tablettenkerne, die mit einer Masse von 1248 mg je Einheit einen Wirkstoffanteil von 80% besitzen. Überraschenderweise zeigen diese Tablettenkerne trotz des hohen Wirkstoffanteils ausgezeichnete mechanische Eigenschaften sowie einen schnellen und vollständigen Zerfall als eine wichtige Voraussetzung für die Arzneistofffreisetzung nach Auflösen des Filmüberzugs. Die Festigkeit der Komprimate lässt sich durch die Bestimmung der Bruch- und Abriebfestigkeit beurteilen. Beide Eigenschaften werden bevorzugt nach den Methoden 2.9.7 und 2.9.8 des europäischen Arzneibuchs bestimmt. Die Bruchfestigkeit der erfindungsgemäßen Mesalazin Hochdosistabletten liegt bei über 160 N, die Abriebfestigkeit bzw. der Abriebverschleiß bei weniger als 1%. Die Zerfallszeit der Tablettenkerne in 0,3 molarem Phosphatpuffer pH 6,8, bevorzugt bestimmt nach der Methode 2.9.1 des europäischen Arzneibuchs, liegt bei weniger als 15 Minuten.

Überraschenderweise kommt der Tablettenkern der erfindungsgemäßen Anmeldung mit weniger Hilfsstoffen aus als bekannte Rezepturen des Tablettenkerns wie z.B. diejenige von Salofalk® 500 mg magensaftresistente Tabletten ohne dabei die Eigenschaften des Formlings zu verändern. Das Wirkstoffgranulat der 1000 mg-Hochdosistabletten, lediglich bestehend aus Mesalazin und Povidon K25, führt dabei zu den identischen biopharmazeutischen und therapeutischen Eigenschaften wie das wesentlich komplexer aufgebaute Granulat von Salofalk® 500 mg magensaftresistente Tabletten, das zur Lösungsvermittlung des Wirkstoffes zusätzlich noch die Hilfsstoffe Natriumcarbonat und Glycin enthält. Diese beiden Bestandteile können erfindungsgemäß in der bevorzugten Ausführungsform ersatzlos entfallen. Überraschenderweise wurde nämlich bei der vorliegenden Erfindung erkannt, dass auf die Verwendung von Natriumcarbonat und Glycin für die vollständige, pH-gesteuerte Freisetzung und Verfügbarkeit verzichtet werden kann. Der Verzicht auf Natriumcarbonat ermöglicht auch, dass das Wirkstoffgranulat der erfindungsgemäßen 1000 mg-Hochdosistablette rein wässrig und nicht unter Verwendung eines organischen Lösungsmittels wie Ethanol hergestellt werden kann. Die Wirksamkeit der vorliegenden Hochdosistablette in der Anwendung wurde durch eine vergleichende, klinische Studie bestätigt.

Neben der verbesserten Granulatrezeptur und Produktform ist als weiteres, entscheidendes und überraschendes Merkmal der Erfindung die optimierte qualitative und quantitative Zusammensetzung des Filmüberzugs der Mesalazin 1000 mg-Hochdosistablette zu nennen. Der Prozess der Befilmung wird dadurch deutlich vereinfacht und beschleunigt, ohne das gewünschte Freisetzungsverhalten des Wirkstoffes zu beeinträchtigen. Damit wird gewährleistet, dass bei vereinfachtem Dosierungsschema sowie erhöhter Patientenakzeptanz die gleiche therapeutische Wirksamkeit wie mit der Einnahme der doppelten Anzahl an Salofalk® 500 mg magensaftresistenten Tabletten erzielt wird.

Es ist bekannt, dass die erfolgreiche Behandlung des akuten Schubs der Colitis ulcerosa sowie die Rezidivprophylaxe mit Salofalk® 500 mg magensaftresistenten Tabletten ein definiertes Freisetzungsprofil von Mesalazin aus der Arzneiform erfordern. Die lokale Verfügbarkeit des Wirkstoffes startet nach der Magenpassage mit einer weiteren zeitlichen Verzögerung von mindestens 15 Minuten im Dünndarm. Der Filmüberzug muss also einerseits die Magensaftresistenz der Darreichungsform sicherstellen und darf andererseits erst mindestens 15 Minuten nach der Magenpassage im Dünndarm vollständig aufgelöst sein, um den Start der Wirkstofffreisetzung am gewünschten Zielort zu gewährleisten. Bei Salofalk® 500 mg magensaftresistenten Tabletten wird dieses Freisetzungsverhalten durch den aufeinanderfolgenden Auftrag von drei Schichten von insgesamt 90,5 mg Methacrylsäure-Methylmethacrylat-Copolymer (1:1) je Tablette bezogen auf eine Oberfläche von ca. 3,7 cm² (entsprechend 25 mg/cm² und einem Auftrag von 13%) erzielt. Es handelt sich hierbei um ein anionisches Polymer aus Methacrylsäure und Methylmethacrylat mit dem Handelsname Eudragit® L. Das Verhältnis der freien Carboxygruppen zu den Estergruppen ist 1:1 und bestimmt die Löslichkeit des Polymers ab einem pH-Wert von 6,0. In Verbindung mit der aufgetragenen Filmmenge werden dadurch die Magensaftresistenz sowie der Start der Wirkstofffreisetzung am Zielort sichergestellt. Die Tablettenkerne werden vor dem Aufbringen des Methacrylsäure-Methylmethacrylat-Copolymer (1:1) noch mit einer nicht-funktionalen Hypromellose-Schicht isoliert.

Die erfindungsgemäß bevorzugt eingesetzte "Grundierschicht", die bevorzugt aus einem nicht enterischen Beschichtungsstoff, wie Hydroxypropylmethylcellulose, besteht, löst sich unabhängig vom pH-Wert im Darm auf, nachdem die enterische äußere Beschichtung aufgelöst wurde. Durch diese Grundierschicht wird die Freisetzung des Wirkstoffes jedoch nicht beschleunigt oder in anderer Weise modifiziert.

Die erfindungsgemäße 1000 mg-Hochdosistablette gewährleistet das erforderliche Freisetzungsverhalten von Mesalazin im Dünndarm, indem die Tabletten mit deutlich weniger Polymermaterial überzogen werden müssen. Im Gegensatz zu Salofalk® 500 mg magensaftresistenten Tabletten umfasst der Überzug lediglich 72,0 mg an Filmbildner je Tablette bezogen auf eine Oberfläche von ca. 5,3 cm² (entsprechend 14 mg/cm² und einem Auftrag von 6%). Diese deutliche Reduktion der Filmmenge ist verbunden mit einer optimierten qualitativen Zusammensetzung des Überzugmaterials für die verzögerte Wirkstofffreisetzung, das anstatt in drei nur noch in zwei Schichten aufgetragen werden muss. Als Filmbildner werden dabei zwei Copolymerisate vom Eudragit®-Typ verwendet. Neben Eudragit® L wird eine Mischung aus Eudragit L® und Eudragit® S verwendet. Eudragit® S ist der Handelsname für ein anionisches Polymer aus Methacrylsäure und Methylmethacrylat, wobei das Verhältnis der freien Carboxygruppen zu den Estergruppen 1:2 beträgt. Die Löslichkeit dieses Copolymers ist ab einem pH-Wert von 7,0 gegeben.

Wie in Beispiel 1 aufgeführt, werden die mit Hypromellose isolierten Tablettenkerne in der bevorzugten Ausführungsform zunächst mit einer Schicht Methacrylsäure-Methylmethacrylat-Copolymer (1:1) überzogen. Dabei werden 47,2 mg des Polymers auf die Oberfläche jedes Tablettenkerns aufgebracht (entsprechend 9 mg/cm² und einem Auftrag von 4%). Anschließend erfolgt mit einer zweiten Schicht der Auftrag einer Mischung bestehend aus 60 Gew.-% Methacrylsäure-Methylmethacrylat-Copolymer (1:1) und 40 Gew.-% Methacrylsäure-Methylmethacrylat-Copolymer (1:2). Der aus dieser Mischung resultierende Film überzieht jede Tablettenoberfläche mit weiterem Polymermaterial (entsprechend 5 mg/cm² und einem Auftrag von nur etwa 2%) bestehend aus Eudragit L® und Eudragit® S.

Durch die optimierte qualitative und quantitative Zusammensetzung des Filmüberzugs der Mesalazin 1000 mg-Hochdosistablette wird die Magensaftresistenz sowie das gewünschte Freisetzungsverhalten im oberen Dünndarm durch einen deutlich geringeren Auftrag an Polymermaterial realisiert. Damit ist ein wesentlich einfacheres Konzept der Befilmung der Tablettenkerne gegeben. Vergleichende Untersuchungen zur Wirkstofffreisetzung *in-vitro* zeigen identische Auflösungsprofile zwischen der erfindungsgemäßen Hochdosistablette sowie der Salofalk 500 mg magensaftresistenten Tabletten im künstlichen Darmsaft mit der geforderten Verzögerungszeit von mindestens 15 Minuten in vitro. Die Magensaftresistenz ist ebenfalls sichergestellt. Beispiel 3 zeigt die Ergebnisse der vergleichenden Wirkstofffreisetzungsversuche.

Die Durchführung von vergleichenden Wirkstofffreisetzungsversuchen, wie in Beispiel 3 gezeigt, ist ein wichtiges Hilfsmittel zur Formulierungsauswahl, da die Ergebnisse zur Vorhersage der biopharmazeutischen und therapeutischen Eigenschaften herangezogen werden können. Wenn sich zwei zu vergleichende Formulierungen in diesen Versuchen ähnlich verhalten, sind Rückschlüsse auf die tatsächliche *in-vivo* Situation im Patienten möglich.

Die standardmäßig verwendeten *in-vitro* Freisetzungsapparaturen, wie die Drehkörbchen- und Blattrührerapparatur, sind nur bedingt geeignet, da sie relevante Bedingungen der Gastrointestinalpassage nicht in geeigneter Weise nachstellen können. Dazu gehören unterschiedliche Passage- und Verweilzeiten, Zusammensetzung und Volumen der gastrointestinalen Flüssigkeit sowie hydrodynamische Bedingungen. Je genauer die gastrointestinalen Segmente *in vitro* simuliert werden, desto besser sind die Vorhersagen über das tatsächliche Verhalten der Arzneiform *in vivo.*

Das weitaus bessere Verfahren zur Vorhersage des Produktverhaltens unter den Bedingungen nach der Einnahme durch den Patienten ist die möglichst getreue Simulation der Gastrointestinalpassage. Besonders geeignet hierfür ist die Apparatur des eintauchenden Zylinders (Apparatur 3 gemäß Kapitel 2.9.3 des Europäischen Arzneibuchs). Dieses System erlaubt die Charakterisierung und den Vergleich von Arzneiformen unter Berücksichtigung biorelevanter Prüfbedingungen. Dazu gehören der mehrfache Medienwechsel, die Verwendung eines kleineren Prüfvolumens sowie das Nachstellen von Transportbewegungen. Beispiel 6 zeigt die Ergebnisse der Wirkstofffreisetzungsversuche der erfindungsgemäßen 1000 mg-Hochdosistablette im Vergleich mit Salofalk 500 mg magensaftresistenten Tabletten unter Verwendung der Apparatur des eintauchenden Zylinders.

In einer besonders bevorzugten Ausführungsform weist eine erfindungsgemäße Hochdosistablette mit 1000 mg Mesalazin ein Freisetzungsprofil des Wirkstoffes im Biodissolution-Test, wie in Beispiel 6 gezeigt, auf, das im Wesentlichen dem Freisetzungsprofil von zwei 500 mg Tabletten entspricht. Das bedeutet, dass bis zu 120 min weniger als 10 % Wirkstoff gelöst sind und, dass nach 180 min 70 % oder bevorzugt mind. 80% mehr Wirkstoff gelöst sind.

In einer weiteren bevorzugten Ausführungsform sind die Hochdosistabletten oblong mit parallelen Längsseiten und gerundeten Schmalseiten. Die Oberflächen sind bikonvex gewölbt und frei von Kerben oder Bruchrillen. Die Tablettenhöhe h liegt hierbei in dem Bereich von 6 bis 8 mm, bevorzugt in dem Bereich von 6,8 mm bis 7,4 mm, besonders bevorzugt bei 7,1 mm. Die Tablettenlänge l liegt hierbei in dem Bereich von 19 bis 22 mm, bevorzugt in dem Bereich von 20 mm bis 21 mm, besonders bevorzugt in dem Bereich von 20,1 mm bis 20,6 mm, wie zum Beispiel 20,3 mm. Die Tablettenbreite b liegt hierbei in dem Bereich von 8 bis 10 mm, bevorzugt in dem Bereich von 9 mm bis 9,8 mm, besonders bevorzugt in dem Bereich von 9,2 mm bis 9,7 mm, wie zum Beispiel 9,4 mm.

Die Wölbungsradien (Doppelwölbung) der konvexen Ober-und Unterseite der Oblongtablette betragen 4,25 mm und 60,00 mm in Längsrichtung sowie 4,25 mm und 8,00 mm in Querrichtung und ermöglichen eine optimale Befilmung der Tabletten sowie verbesserte Schluckbarkeit der Filmtabletten.

Beispiel 2 beschreibt die Abmessungen der erfindungsgemäßen Mesalazin 1000 mg-Hochdosistabletten. Die Tablettenoberfläche beträgt ca. 5 cm². Das gewählte Tablettenformat führt zu stabilen Formlingen mit optimalen mechanischen und geometrischen Eigenschaften für die Auftragung von gleichmäßigen und dünnen Filmüberzügen im Trommelcoater. Gleichzeitig werden durch die Form sowie die Abmessungen die Schluckbarkeit und damit die Akzeptanz der Tabletten durch die Patienten im hohen Maße gesteigert.

Die beschriebenen Merkmale der Erfindung führen zur Bereitstellung einer stabilen Mesalazin 1000 mg-Hochdosistablette. Die Primärverpackung der Tabletten erfolgt bevorzugt in Durchdrückpackungen (Blister) bestehend aus PVC oder aus mit PVDC-beschichtetem PVC Kunststoffformfolien und aus Hart-Aluminium-Deckfolien. Die qualitative und quantitative Zusammensetzung sowie das gewählte Herstellungsverfahren und die Auswahl des Primärpackmittels stellen sicher, dass die Tabletten bei Lagerung unter den Bedingungen der Klimazone II keine Veränderungen über einen Zeitraum von mindestens 36 Monate zeigen. Auch unter den Bedingungen von Belastungsversuchen sind die Tabletten stabil. Ergebnisse von Haltbarkeitsversuchen sind in Beispiel 4 aufgeführt.

Desweiteren betrifft die vorliegende Erfindung die Verwendung von Mesalazin-haltigen Hochdosistabletten wie oben beschrieben in der Behandlung von chronisch entzündlichen Darmerkrankungen. In einer Ausführungsform wird die erfindungsgemäße Mesalazin 1000 mg-Hochdosistablette wie Salofalk® 500 mg magensaftresistente Tabletten in der Behandlung des akuten Schubs der Colitis ulcerosa sowie zur Rezidivprophylaxe eingesetzt.

In einer klinischen Studie mit dem Ziel, die Vergleichbarkeit der erfindungsgemäßen Hochdosistablette mit den im Stand der Technik beschriebenen 500 mg Tabletten zu zeigen, wurden beide Formulierungen über einen Zeitraum von 8 Wochen getestet. Insgesamt 306 Patienten mit einer aktiven Colitis Ulcerosa wurden in einer doppel-blind, "double-dummy" durchgeführten klinischen Studie in mehreren europäischen Ländern mit entweder der erfindungsgemäßen 1000 mg Mesalazin-Tablette (3x tägliche Einnahme) oder 2x500 mg Mesalazin-Tabletten (ebenfalls 3x tägliche Einnahme) behandelt. Als wirksam behandelt wurden solche Patienten gewertet, die nach 8 Wochen Behandlung (Visite 4 der Patienten) eine klinische Remission erreichten. Klinische Remission wurde sehr stringent definiert als einen Krankheitsaktivitätsindex ≤4 (CAI, Colitis Activity Index) mit einer täglichen Stuhlfrequenz in diesem Index von 0 (definiert als < 18 Stühle pro Woche) und einem Unterscore für rektale Blutung ebenfalls von 0 (definiert als 0-1 blutigen Stuhl in diesem Unterscore). Beispiel 5 zeigt die Ergebnisse dieser Studie in den verschiedenen statistischen Auswertepopulationen. Die klinische Studie konnte aufgrund der vergleichbaren Wirksamkeit bereits nach der Zwischenanalyse gestoppt werden. In der Behandlungsgruppe, die die erfindungsgemäße Hochdosistablette eingenommen hatten (M1000), erreichten 46,6% der Patienten den primäre Wirksamkeitsnachweis, im Vergleich zu 38,6% der Patienten in der Behandlungsgruppe mit 2x500 mg Mesalazintabletten (M2x500) täglich (Beispiel 5).

Die therapeutische Wirksamkeit, die Sicherheit und die Verträglichkeit von Mesalazin werden durch beide Formulierungen gleichermaßen gewährleistet. Es besteht hinsichtlich dieser Kriterien kein Unterschied zwischen der Salofalk® 500 mg magensaftresistente Tabletten und der erfindungsgemäßen Mesalazin 1000 mg-Hochdosistablette.

Allerdings bevorzugen die Patienten eindeutig die medikamentöse Behandlung mit der Mesalazin 1000 mg-Hochdosistablette. Eine große Mehrheit der Patienten (47,7%) spricht sich deutlich für die Einnahme von nur einer Tablette aus. Lediglich 10,5% der an der Studie teilnehmenden Patienten bevorzugte die Einnahme von zwei kleineren Mesalazin-Tabletten. Das Ergebnis ist deshalb robust, da alle Patienten in dieser doppelblinden, klinischen Studie beide Darreichungsformen, entweder als Placebo oder als Verum während der Studiendauer eingenommen haben. Dieses überraschend klare Votum ist auf die gewählte Formgebung und Größe der Hochdosistablette und damit auf ein wesentlich vereinfachtes und patientenfreundlicheres Dosierungs- und Verabreichungsschema zurückzuführen, wodurch die regelmäßige Medikamenteneinnahme erleichtert und die Akzeptanz durch den Patienten erhöht wird. Demzufolge kann mit der erfindungsgemäßen Mesalazin 1000 mg-Hochdosistablette die Therapietreue (Compliance) gesteigert werden, so dass sich der Therapieerfolg von Mesalazin im Vergleich mit den getesteten, etablierten Darreichungsformen sogar prozentual höher erzielen lässt. Therapie- und patientenbedingte Faktoren als Ursachen für eine mangelnde Therapietreue können daher mit der erfindungsgemäßen Hochdosistablette deutlich reduziert werden.

Bevorzugte Ausführungsformen der vorliegenden Erfindung werden durch die nachfolgenden Beispiele verdeutlicht.

### Beispiel 1: Qualitative und quantitative Zusammensetzung der bevorzugten Ausführungsformen von Mesalazin magensaftresistente 1000 mg-Hochdosistablette

**Tabelle 1**

| **Bestandteil** | **Masse je Tablette** | |
|---|---|---|
| Mesalazin | 1000,00 | mg |
| Povidon K25 | 70,00 | mg |
| Mikrokristalline Cellulose | 100,00 | mg |
| Wasserfreies hochdisperses Siliziumdioxid | 5,00 | mg |
| Croscarmellose-Natrium | 60,00 | mg |
| Calciumstearat | 13,00 | mg |
| Hypromellose | 6,50 | mg |
| Macrogol 6000 | 7,60 | mg |
| Methacrylsäure-Methylmethacrylat-Copolymer 1:1 | 57,10 | mg |
| Methacrylsäure-Methylmethacrylat-Copolymer 1:2 | 14,90 | mg |
| Talkum | 12,05 | mg |
| Titandioxid | 0,60 | mg |
| Eisenoxid, gelb | 0,75 | mg |
| | | |
| Filmtablettenmasse | 1347,50 | mg |

Die Zusammensetzung einer bevorzugten 1000 mg Hochdosistablette ist in Tabelle 1 wiedergegeben.

Die obigen Bestandteile des Tablettenkerns: Mesalazin, Povidon K25, mikrokristalline Cellulose, wasserfreies hochdisperses Siliziumdioxid, Croscarmellose-Natrium und Calciumstearat summieren sich auf 1248 mg. Dies stellt 80,1 Gew.-% der gebrauchsfertigen Tablette dar. Der Anteil der Beschichtungen beträgt damit weniger als 20,0 Gew.-% der fertigen Tablette.

### Beispiel 2: Tablettenform, Tablettengröße, Tablettengeometrie sowie Tablettenabmessungen von Mesalazin magensaftresistente 1000 mg-Hochdosistablette

### Tablettengeometrie

| | |
|---|---|
| • Form, Aussehen | Oblong, stäbchenförmig |
| • Seiten | Parallele bis leicht gerundete Längsseiten und gerundete Schmalseiten |
| • Oberfläche | Bikonvex gewölbte Oberflächen, frei von Kerben oder Bruchrillen |

### Tablettengröße und -abmessungen (ohne Filmüberzug)

| | |
|---|---|
| • Tablettenhöhe | 7,1 mm (6,8 mm bis 7,4 mm) |
| • Tablettenlänge | 20,3 mm (20,1 mm bis 20,6 mm) |
| • Tablettenbreite | 9,4 mm (9,2 mm bis 9,7 mm) |
| • Steghöhe | 3,2 mm (2,9 mm bis 3,5 mm) |
| • Kalottenhöhe | 1,95 mm |
| • Umfang | 5,1 cm |
| • Mantel- bzw. Stegfläche | 1,64 cm² |
| • Pressfläche | 1,84 cm² |
| • Oberfläche | 5,32 cm² (5,16 cm² bis 5,47 cm²) |

### Tablettenwölbung (Wölbungsradien der Kalotte)

• Wölbungs-, Krümmungsradien längs

| | |
|---|---|
| - Radius 1: | 4,25 mm |
| - Radius 2: | 60,00 mm |

• Wölbungs-, Krümmungsradien quer

| | |
|---|---|
| - Radius 1: | 4,25 mm |
| - Radius 2: | 8,00 mm |

### Beispiel 3: Nachweis äquivalenter Wirkstofffreisetzungsprofile von Mesalazin magensaftresistente 1000 mg-Hochdosistablette und Salofalk® 500 mg magensaftresistente Tabletten

Eine erfindungsgemäße Hochdosistablette (1000 mg) zeigt ein äquivalentes Auflösungsprofil von Mesalazin wie zwei Salofalk® 500 mg magensaftresistente Tabletten. Die vergleichenden Untersuchungen zur Wirkstofffreisetzung *in vitro* wurden dabei mit zwei unterschiedlichen Chargen von Mesalazin magensaftresistente 1000 mg-Hochdosistablette in der bevorzugten Ausführungsform durchgeführt und schlossen die Prüfung über zwei Stunden im künstlichen Magensaft mit einem pH-Wert von 1 (0,1 M HCl) sowie die anschließende Prüfung über 60 Minuten im künstlichen Darmsaft mit einem pH-Wert von 6,8 (0,3 M Phosphatpuffer) ein. Nach der Exposition im künstlichen Magensaft zeigten die Filmtabletten der Referenzzubereitung, d.h. Salofalk® 500 mg magensaftresistente Tabletten, und die Filmtabletten der Erfindung, d.h. Mesalazin magensaftresistente 1000 mg-Hochdosistablette, identische Auflösungsprofile von Mesalazin im künstlichen Darmsaft. Nach der geforderten Verzögerungszeit von mindestens 15 Minuten verlaufen die Freisetzungsprofile annähernd deckungsgleich und nach 60 Minuten ist die vollständige Auflösung von Mesalazin erreicht. Die nach den einzelnen Prüfzeitpunkten im künstlichen Darmsaft freigesetzten Mesalazinmengen unterscheiden sich zwischen der Referenzzubereitung und der erfindungsgemäßen Hochdosistablette um weniger als 10%. Gemäß der Leitlinie der Europäischen Arzneimittelagentur (EMA) zu Bioäquivalenzuntersuchungen (CPMP/EWP/QWP/1401/98 Rev. 1/Corr.) lässt sich aus den Ergebnissen der vergleichenden Wirkstofffreisetzungsversuche ein Ähnlichkeitsfaktor (f2) von größer als 50 berechnen. Die Profile gelten damit als identisch. Die folgenden Ausführungen fassen die Versuche im Einzelnen zusammen.

### a) Prüfmuster

- Mesalazin magensaftresistente 1000 mg-Hochdosistablette, Chargen G0605B001, G0605B002
- Salofalk® 500 mg magensaftresistente Tabletten, Charge 1204966001

### b) Parameter der Wirkstofffreisetzungsprüfung

| | |
|---|---|
| • Freisetzungsapparatur: | Blattrührerapparatur (Apparatur 2 gemäß Kapitel 2.9.3 des Europäischen Arzneibuchs) |
| • Prüfmedien | |
| - Künstlicher Magensaft: | 0,1 M HCl, Volumen 500 ml, 120 Minuten |
| - Künstlicher Darmsaft: | 0,3 M Phosphatpuffer pH 6,8, Volumen 1000 ml, 60 Minuten |
| • Temperatur | 37,0°C ± 0,5°C |
| • Rührgeschwindigkeit: | 100 UpM |
| • Anzahl Proben je Prüfzeitpunkt und Charge: | |
| | N = 12 |
| • Prüfzeitpunkte: | 0, 5, 10, 15, 20, 25, 30, 35, 45, 60 Minuten |

### c) Gehaltsbestimmungsmethode für Mesalazin

Der Gehalt der im Prüfmedium freigesetzten Mesalazinmenge erfolgte spektralphotometrisch bei einer Wellenlänge von 370 nm.

### d) Ergebnisse

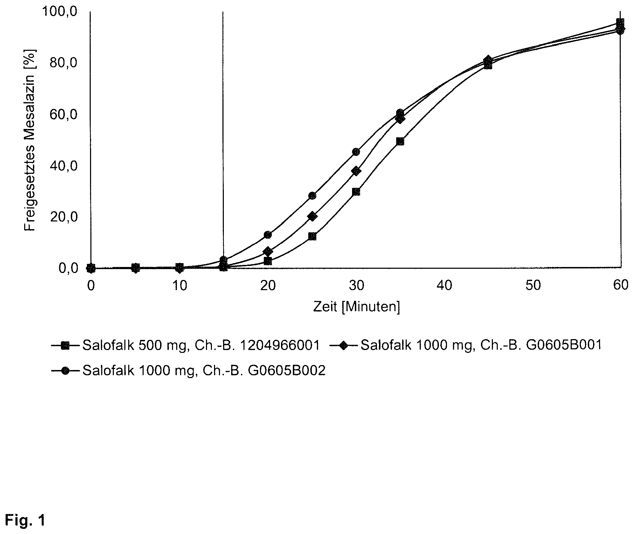

Die Figur 1 zeigt die Freisetzungsprofile der drei getesteten Chargen.

Die Berechnung des Ähnlichkeitsfaktors (*f2*) zum letzten Prüfzeitpunkt ergab folgende Ergebnisse:
- Mesalazin magensaftresistente 1000 mg Hochdosistablette, Charge G0605B001, und Salofalk 500 mg magensaftresistente Tablette, Charge 1204966001: ***f2*** = 66
- Mesalazin magensaftresistente 1000 mg Hochdosistablette, Charge G0605B002, und Salofalk 500 mg magensaftresistente Tablette, Charge 1204966001: ***f2*** = 53

### Beispiel 4: Ergebnisse von Haltbarkeitsuntersuchungen von Mesalazin magensaftresistente 1000 mg-Hochdosistablette

Zwei Chargen von Mesalazin magensaftresistenten 1000 mg-Hochdosistabletten wurden in der bevorzugten Ausführungsform hergestellt, in Durchdrückpackungen (Blister) bestehend aus PVDC/PVC-Aluminium-Folien verpackt und bei 25°C/60 % relativer Feuchte sowie bei 40°C/75% relativer Feuchte für Haltbarkeitsuntersuchungen eingelagert. Nach der Herstellung sowie in regelmäßigen Intervallen während der Lagerung wurden sowohl der Gehalt und die Reinheit der Filmtabletten sowie die Wirkstofffreisetzung bestimmt. Die Gehalts- und Reinheitsbestimmung erfolgten dabei mit einer validierten HPLC/UV-Methode während die Bestimmung der Wirkstofffreisetzung aus den Filmtabletten spektralphotometrisch bei einer Wellenlänge von 370 nm durchgeführt wurde. Die erfindungsgemäße Hochdosistablette zeigt weder unter Langzeitbedingungen (36 Monate bei 25°C/60% relative Feuchte) noch unter beschleunigen Bedingungen (6 Monate bei 40°C/75% relativ Feuchte) Veränderungen in den geprüften Eigenschaften. Die beiden folgenden Tabellen 2 und 3 fassen die Ergebnisse der Haltbarkeitsuntersuchungen der beiden Chargen zusammen.

**Tabelle 2**

| **Mesalazin magensaftresistente 1000 mg-Hochdosistablette, Charge G0605B001** | | | | | | | |
|---|---|---|---|---|---|---|---|
| **Primärpackmittel: PVC/PVDC-Aluminiumblister¹** | | | | | | | |
| **Prüfparameter** | **Lagerzeit (Monate) bei 25°C/60 % relative Feuchte** | | | | | | |
| | **0** | **6** | **9** | **12** | **18** | **24** | **36** |
| **Gehalt Mesalazin (%)** | 97,0 | 96,6 | 98,5 | 98,0 | 100,0 | 98,9 | 97,5 |
| **Summe an Abbauprodukten (%)** | 0,07 | 0,05 | 0,06 | 0,11 | 0,21 | <0,05 | 0,15 |
| **Wirkstofffreisetzung bei 37°C (%)** | | | | | | | |
| **In 0,1 M HCl (120 Minuten)** | Resistent | Resistent | Resistent | Resistent | Resistent | Resistent | Resistent |
| **In Puffer pH 6,8 (15 Minuten)** | 0,8 | 0,1 | 0,1 | 0,4 | 0,3 | 0,5 | 0,5 |
| **In Puffer pH 6,8 (60 Minuten)** | 93,2 | 94,4 | 84,4 | 95,0 | 91,3 | 92,5 | 92,6 |

| | **Lagerzeit (Monate) bei 40°C/75 % relative Feuchte** | | | | | | |
|---|---|---|---|---|---|---|---|
| | **0** | | **3** | | | **6** | |
| **Gehalt Mesalazin (%)** | 97,0 | | 97,7 | | | 97,0 | |
| **Summe an Abbauprodukten (%)** | 0,07 | | 0,07 | | | 0,09 | |
| **Wirkstofffreisetzung bei 37°C (%)** | | | | | | | |
| **In 0,1 M HCl (120 Minuten)** | Resistent | | Resistent | | | Resistent | |
| **In Puffer pH 6,8 (15 Minuten)** | 0,8 | | 0,5 | | | 0,1 | |
| **In Puffer pH 6,8 (60 Minuten)** | 93,2 | | 93,0 | | | 90,5 | |

| | | | | | | | |
|---|---|---|---|---|---|---|---|
| ¹ PCV/PVDC-Folie (250 µm, 60 g/m², orange), Aluminiumfolie (20 µm) | | | | | | | |

**Tabelle 3**

| **Mesalazin magensaftresistente 1000 mg-Hochdosistablette, Charge G0605B002** | | | | | | | |
|---|---|---|---|---|---|---|---|
| **Primärpackmittel: PVC/PVDC-Aluminiumblister¹** | | | | | | | |
| **Prüfparameter** | **Lagerzeit (Monate) bei 25°C/60 % relative Feuchte** | | | | | | |
| | **0** | **6** | **9** | **12** | **18** | **24** | **36** |
| **Gehalt Mesalazin (%)** | 98,5 | 101,6 | 97,0 | 100,6 | 99,6 | 100,4 | 98,2 |
| **Summe an Abbauprodukten (%)** | 0,07 | 0,13 | 0,21 | 0,05 | 0,22 | 0,06 | 0,15 |
| **Wirkstofffreisetzung bei 37°C (%)** | | | | | | | |
| **In 0,1 M HCl (120 Minuten)** | Resistent | Resistent | Resistent | Resistent | Resistent | Resistent | Resistent |
| **In Puffer pH 6,8 (15 Minuten)** | 3,2 | 2,9 | 2,9 | 4,1 | 3,8 | 2,3 | 3,7 |
| **In Puffer pH 6,8 (60 Minuten)** | 92,3 | 91,4 | 95,2 | 96,9 | 96,3 | 95,4 | 92,5 |

| | **Lagerzeit (Monate) bei 40°C/75 % relative Feuchte** | | | | | | |
|---|---|---|---|---|---|---|---|
| | **0** | | **3** | | | **6** | |
| **Gehalt Mesalazin (%)** | 98,5 | | 96,2 | | | 102,4 | |
| **Summe an Abbauprodukten (%)** | 0,07 | | 0,06 | | | 0,07 | |
| **Wirkstofffreisetzung bei 37°C (%)** | | | | | | | |
| **In 0,1 M HCl (120 Minuten)** | Resistent | | Resistent | | | Resistent | |
| **In Puffer pH 6,8 (15 Minuten)** | 3,2 | | 2,6 | | | 1,3 | |
| **In Puffer pH 6,8 (60 Minuten)** | 92,3 | | 99,5 | | | 93,7 | |

| | | | | | | | |
|---|---|---|---|---|---|---|---|
| ¹ PCV/PVDC-Folie (250 µm, 60 g/m², orange), Aluminiumfolie (20 µm) | | | | | | | |

### Beispiel 5: Klinische Daten

Die Ergebnisse der klinischen Studie sind in der Tabelle 4 zusammengefasst.

| **Analyse-Ansatz** | **Anzahl (%) der Patienten in klini-scher Remission bei V4-LOCF** | | | | | | **Diff^{a}** | **95%-RCI^{b}** | **Sign. level^{c}** | **p-Wert^{d}** |
|---|---|---|---|---|---|---|---|---|---|---|
| | **M1000** | | | **M2x500** | | | | | | |
| | **N** | **n** | **%** | **N** | **n** | **%** | | | | |
| **PP Interim I^{e}** | **103** | **48** | **46.6** | **114** | **44** | **38.6** | **8.0%** | **[-9.6%**, **25.2%]** | **0.0043** | **0.0003** |
| FAS Interim I | 115 | 53 | 46.1 | 123 | 46 | 37.4 | 8.7% | [-8.1%, 25.0%] | 0.0043 | <0.0001 |
| | | | | | | | | | | |
| PP Final^{f} | 134 | 64 | 47.8 | 144 | 61 | 42.4 | 5.4% | [-10.2%, 20.8%] | 0.0043 | 0.0003 |
| FAS Final^{f} | 151 | 68 | 45.0 | 155 | 65 | 41.9 | 3.1% | [-11.7%, 17.8%] | 0.0043 | 0.0006 |

| | | | | | | | | | | |
|---|---|---|---|---|---|---|---|---|---|---|
| ^{a} Differenz zwischen Proportionen (_{πM1000} - _{πM2x500}) ^{b} Wiederholter 95% Konfidenzinterval (RCI) ^{c} Einseitiger lokaler Signifikanz-Level ^{d} Testen von Ho (π_{M1000} - π_{M2x500} ≤ -0.15) durch inverse normale Teststatistik ^{e} Primäranalyse ^{f} PP / FAS Analyse-Ansätze, unter Berücksichtigung von 68 overrunning Patienten, die während der Interim-Analyse I in die Studie aufgenommen wurden | | | | | | | | | | |

In dieser Tabelle 4 beschreibt PP die Per Protocol-Analyse und FAS die Analyse im "Full Analysis Set", wobei die Patienten der PP-Population die Studie protokollgemäß durchgeführt haben und in der FAS-Population alle 306 Patienten ausgewertet wurden, die in diese Studie eingeschlossen wurden.

### Beispiel 6: "Bio-Dissolution Test" der erfindungsgemäßen Hochdosistablette

Um die Abweichungen der Auflösung, die beiden einzelnen Patienten zwangsläufig auftreten, zu objektivieren und zu relativieren, wurde in vitro ein sogenannter "Biodissolution"-Test durchgeführt.

Zum Nachweis der äquivalenten Wirkstofffreisetzungsprofile von Mesalazin magensaftresistente 1000 mg-Hochdosistablette und Salofalk 500 mg magensaftresistente Tabletten unter biorelevanten Prüfbedingungen wurde folgender Test durchgeführt:
Die Verwendung der Apparatur des eintauchenden Zylinders (Apparatur 3 gemäß Kapitel 2.9.3 des Europäischen Arzneibuchs) erlaubt es, die zeitlich verzögerte Freisetzung von Mesalazin aus der erfindungsgemäßen Hochdosistablette sowie aus Salofalk 500 mg magensaftresistenten Tabletten unter biorelevanten Bedingungen zu vergleichen. Dabei befindet sich je eine Dosis der Tabletten in einem Glasröhrchen, das nach oben und unten mit einem Maschendrahtgeflecht verschlossen ist, um die Arzneiform zu halten. Dieser Glaszylinder wird in einem Auflösungsgefäß mit einer Behältergröße von ca. 325 ml auf und ab bewegt. Im Unterschied zu den Standardapparaturen zur Bestimmung der Wirkstofffreisetzung können mit diesem System eine Reihe von Testmedien untersucht werden. Der Glaszylinder bewegt sich in vertikaler Bewegungsrichtung ca. 10 cm im Auflösungsgefäß, das 200 ml Testmedium enthält. Die Geschwindigkeit der Bewegung liegt dabei bei 10 Eintauchbewegungen pro Minute (dips per minute, dpm). Die Vorteile des Systems liegen darin, dass durch einen mehrfachen Medienwechsel im Auflösungsgefäß die Tabletten mit unterschiedlichen Prüfflüssigkeiten in Kontakt treten und damit die Gastrointestinalpassage simuliert werden kann.

Für die Versuche wurden erfindungsgemäße 1000 mg-Hochdosistabletten und Salofalk 500 mg magensaftresistenten Tabletten aus herkömmlichen Produktionschargen herangezogen. Für den Vergleich der beiden Tablettenformen wurden insgesamt fünf Prüfmedien verwendet, die vor allem die pH-Verhältnisse der einzelnen Segmente des Magen-DarmTraktes im nüchternen Zustand nachstellen. Die Verweilzeiten in den einzelnen Medien kombiniert mit den Agitationsbewegungen entsprechen den Bedingungen, denen die Arzneiform auch *in vivo* ausgesetzt wird. Die Versuchsergebnisse zeigen eindrucksvoll, dass die Wirkstofffreisetzung von Mesalazin aus der erfindungsgemäßen Hochdosistablette und aus Salofalk 500 mg magensaftresistent Tablette gleichermaßen erst unter den Bedingungen des distalen Ileums erfolgt. Hier wird der Wirkstoff vollständig aus den Tabletten freigesetzt und ist für die lokale Wirkung verfügbar. Der Wirkstoff wird also durch die Formulierungen an den gewünschten Zielort transportiert. Die folgenden Ausführungen fassen die Versuche im Einzelnen zusammen:

### a) Prüfmuster

- Mesalazin magensaftresistente 1000 mg-Hochdosistablette, Charge 1501215501
- Salofalk 500 mg magensaftresistent Tabletten, Charge 1601218801

### b) Parameter der Wirkstofffreisetzungsprüfung

| | |
|---|---|
| • Freisetzungsapparatur: | Apparatur des eintauchenden Zylinders (Apparatur 3 gemäß Kapitel 2.9.3 des Europäischen Arzneibuchs) |
| • Maschenweite: | 840 µm |
| • Volumen jedes Auflösungsgefäßes: | 200 ml |
| • Temperatur: | 37,0 ± 0,5°C |
| • Eintauchbewegungen: | 10 Eintauchbewegungen pro Minute (dips per minute, dpm) |
| • Anzahl Proben je Prüfzeitpunkt und Charge: | N = 6 |
| • Prüfmedien und Entnahme- bzw. Prüfzeitpunkte: | |

| **Segment des Gastroin-testinaltraktes** | **pH-Wert** | **Prüfmedium** | **Entnahme- und Prüfzeitpunkte (Minuten)** |
|---|---|---|---|
| Magen | 1,2 | 0,1 M HCl | 60 |
| Proximales Jejunum | 6,5 | Phosphatpuffer (Ph. Eur. 5.17.1) | 15 |
| Distales Jejunum | 6,8 | Simulierte Intestinalflüssigkeit ohne Pepsins (SIFₛₚ) (Ph. Eur. 5.17.1) | 15 |
| Proximales Ileum | 7,2 | Phosphatpuffer (Ph. Eur. 5.17.1) | 30 |
| Distales Ileum | 7,5 | Simulierte Intestinalflüssigkeit ohne Pepsins (SIFₛₚ) (Ph. Eur. 5.17.1)¹ | 5, 10, 20, 30, 40, 50, 60, 90, 120 |
| Dickdarm | Nicht geprüft | | |

| | | | |
|---|---|---|---|
| ¹ Berechnete Osmolarität ∼ 0,5 osmol/l | | | |

### c) Gehaltsbestimmung für Mesalazin

Der Gehalt der im Prüfmedium freigesetzten Mesalazinmenge erfolgte durch Hochleistungsflüssigchromatographie mit UV/VIS-Detektion (HPLC/UV). Die Detektionswellenlänge betrug 220 nm.

### d) Ergebnisse

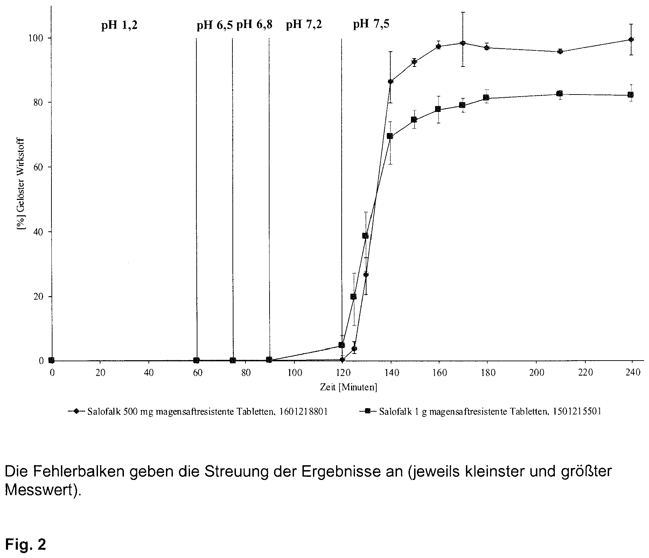

Die Figur 2 zeigt die Freisetzungsprofile der beiden getesteten Chargen.

## Patentansprüche

1. Orale magensaftresistente Hochdosistablette, umfassend als aktiven Bestandteil 1000 mg Mesalazin oder ein pharmazeutisch verträgliches Salz davon und zumindest einen Hilfsstoff, der keine Matrix-bildende Bestandteile enthält, wobei die Masse der Hochdosistablette maximal 35 % höher als die Masse des aktiven Bestandteils ist und der zumindest eine Hilfsstoff aus Polyvinylpyrrolidon besteht, wobei die Hochdosistablette zumindest einen Filmüberzug aufweist, der ein magensaftresistentes Polymer umfasst, und der weniger als 10 Gew.-% bezogen auf das Gesamtgewicht der Hochdosistablette ausmacht,

2. Orale magensaftresistente Hochdosistablette gemäß Anspruch 1, **dadurch gekennzeichnet, dass** der Anteil an dem zumindest einen Hilfsstoff maximal 30 Gew.-% bezogen auf das Gesamtgewicht der Hochdosistablette ausmacht.

3. Orale magensaftresistente Hochdosistablette gemäß einem der Ansprüche 1 bis 2, **dadurch gekennzeichnet, dass** sie 50 bis 100 mg Povidon enthält.

4. Orale magensaftresistente Hochdosistablette gemäß einem der vorherigen Ansprüche, wobei die Tablette eine oblonge Form mit parallelen Längsseiten und gerundeten Schmalseiten aufweist und die Oberflächen bikonvex gewölbt und frei von Kerben oder Bruchrillen sind.

5. Orale magensaftresistente Hochdosistablette gemäß einem der Ansprüche 1-4, **dadurch gekennzeichnet, dass** das Freisetzungsprofil im Biodissolutions-Test des Wirkstoffes Mesalazin einer Hochdosistablette im Wesentlichen dem Freisetzungsprofil von zwei Tabletten entspricht, die jeweils die halbe Menge des Wirkstoffes der Hochdosistablette enthalten.

6. Orale magensaftresistente Hochdosistablette gemäß Anspruch 1, **dadurch gekennzeichnet, dass** sie eine Grundierschicht aufweist, die die Freisetzung des Wirkstoffes nicht beschleunigt oder in anderer Weise modifiziert.

7. Orale magensaftresistente Hochdosistablette gemäß einem der vorherigen Ansprüche zur Verwendung bei der Behandlung von chronisch entzündlichen Darmerkrankungen.

8. Orale magensaftresistente Hochdosistablette zur Verwendung gemäß Anspruch 7 bei der Behandlung von Colitis ulcerosa.

9. Orale magensaftresistente Hochdosistablette zur Verwendung gemäß einem der Ansprüche 7 oder 8 bei der Behandlung von Colitis ulcerosa in der Remissionsphase.

10. Orale magensaftresistente Hochdosistablette zur Verwendung gemäß einem der Ansprüche 7 bis 9 zur Behandlung von chronischen entzündlichen Darmerkrankungen, wobei täglich drei Hochdosistabletten verabreicht werden.

11. Orale magensaftresistente Hochdosistablette zur Verwendung gemäß Anspruch 10, wobei je eine Hochdosistablette morgens, mittags und abends verabreicht wird.

## Claims

1. An oral enteric high-dose tablet comprising as an active ingredient 1000mg of mesalazine or a pharmaceutically acceptable salt thereof and at least one excipient that does not contain any matrix-forming ingredients, wherein the mass of the high-dose tablet is at most 35% higher than the mass of the active ingredient and the at least one excipient consists of polyvinylpyrrolidone, wherein the high-dose tablet has at least one film coating that comprises an enteric polymer and that makes up less than 10% by weight based on the total weight of the high-dose tablet.

2. The oral enteric high-dose tablet according to claim 1, **characterized in that** the proportion of the at least one excipient makes up at most 30% by weight based on the total weight of the high-dose tablet.

3. The oral enteric high-dose tablet according to any one of claims 1 to 2, **characterized in that** it contains 50 to 100mg of povidone.

4. The oral enteric high-dose tablet according to any one of the preceding claims, wherein the tablet has an oblong shape with parallel longitudinal sides and rounded narrow sides and the surfaces are curved biconvex and free from notches or break grooves.

5. The oral enteric high-dose tablet according to any one of claims 1-4, **characterized in that** the release profile in the biodissolution test of the active ingredient mesalazine of a high-dose tablet substantially corresponds to the release profile of two tablets each containing half the amount of the active ingredient of the high-dose tablet.

6. The oral enteric high-dose tablet according to claim 1, **characterized in that** it has a priming coat that does not accelerate or otherwise modify the release of the active ingredient.

7. The oral enteric high-dose tablet according to any one of the preceding claims for use in the treatment of chronical inflammatory bowel diseases.

8. The oral enteric high-dose tablet for use according to claim 7 in the treatment of ulcerative colitis.

9. The oral enteric high-dose tablet for use according to any one of claims 7 or 8 in the treatment of ulcerative colitis in the remission phase.

10. The oral enteric high-dose tablet for use according to any one of claims 7 to 9 for the treatment of chronical inflammatory bowel diseases, wherein three high-dose tablets daily are administered.

11. The oral enteric high-dose tablet for use according to claim 10, wherein one high-dose tablet each is administered in the morning, at noon, and in the evening.

## Revendications

1. Comprimé hautement dosé gastro-résistant à administration orale, comprenant comme composant actif 1000 mg de mésalazine ou un sel pharmaceutiquement acceptable de celle-ci et au moins un excipient, qui n'inclut pas de composants formant matrice, dans lequel la masse du comprimé hautement dosé est au maximum à 35 % supérieure à la masse du composant actif et l'au moins un excipient est constitué par du polyvinylpyrrolidone, dans lequel le comprimé hautement dosé présente au moins un enrobage pelliculé, qui comprend un polymère gastro-résistant, et qui représente moins de 10 % en poids par rapport au poids total du comprimé hautement dosé.

2. Comprimé hautement dosé gastro-résistant à administration orale selon la revendication 1, **caractérisé en ce que** la part de l'au moins un excipient représente au maximum 30 % en poids par rapport au poids total du comprimé hautement dosé.

3. Comprimé hautement dosé gastro-résistant à administration orale selon l'une quelconque des revendications 1 à 2, **caractérisé en ce qu'**il inclut 50 à 100 mg de povidone.

4. Comprimé hautement dosé gastro-résistant à administration orale selon l'une quelconque des revendications précédentes, dans lequel le comprimé présente une forme oblongue avec des côtés longs parallèles et des côtés étroits arrondis et les surfaces sont incurvées de manière biconvexe et sont exemptes d'entailles ou de rainures de fragmentation.

5. Comprimé hautement dosé gastro-résistant à administration orale selon l'une quelconque des revendications 1 à 4, **caractérisé en ce que** le profil de libération dans le test de biodissolution du principe actif mésalazine d'un comprimé hautement dosé correspond sensiblement au profil de libération de deux comprimés, qui contiennent respectivement la moitié de la quantité du principe actif du comprimé hautement dosé.

6. Comprimé hautement dosé gastro-résistant à administration orale selon la revendication 1, **caractérisé en ce qu'**il présente une couche primaire qui n'accélère ni ne modifie d'une autre manière la libération du principe actif.

7. Comprimé hautement dosé gastro-résistant à administration orale selon l'une quelconque des revendications précédentes à utiliser pour le traitement de maladies intestinales inflammatoires chroniques.

8. Comprimé hautement dosé gastro-résistant à administration orale à utiliser selon la revendication 7 pour le traitement de la colite ulcéreuse.

9. Comprimé hautement dosé gastro-résistant à administration orale à utiliser selon l'une quelconque des revendications 7 ou 8 pour le traitement de la colite ulcéreuse en phase de rémission.

10. Comprimé hautement dosé gastro-résistant à administration orale à utiliser selon l'une quelconque des revendications 7 à 9 pour le traitement de maladies intestinales inflammatoires chroniques, dans lequel trois comprimés hautement dosés sont administrés chaque jour.

11. Comprimé hautement dosé gastro-résistant à administration orale à utiliser selon la revendication 10, dans lequel respectivement un comprimé hautement dosé est administré le matin, à midi et le soir.
